(19) Europäisches Patentamt

European Patent Office

Office européen des brevets

(11) **EP 1 074 254 A2**

(12) **EUROPEAN PATENT APPLICATION**

(43) Date of publication:
**07.02.2001 Bulletin 2001/06**

(51) Int. Cl.⁷: **A61K 31/385**, A61K 31/35,
A61K 31/12, A61K 31/015

(21) Application number: **00115505.0**

(22) Date of filing: **19.07.2000**

(84) Designated Contracting States:
**AT BE CH CY DE DK ES FI FR GB GR IE IT LI LU
MC NL PT SE**
Designated Extension States:
**AL LT LV MK RO SI**

(30) Priority: **20.07.1999 EP 99830464**

(71) Applicant:
**MEDIS S.r.l. Medical Infusion Systems
20127 Milano (IT)**

(72) Inventor: **Ghisalberti, Carlo
20154 Milano (IT)**

(74) Representative:
**Minoja, Fabrizio, Dr.
Bianchetti Bracco Minoja S.r.l.
Via Rossini, 8
20122 Milano (IT)**

(54) **Use of plant polyphenols with vitamines for treating iron overload**

(57) The present invention relates to the use of anthocyanins or proantocyanidins in combination with lipophilic antioxidant vitamins for the treatment of the iron overloading conditions. Also claimed are compositions comprising anthocyanins or proantocyanidins and lipophilic antioxidant vitamins, preferably combined with selected pro-antioxidant vitamins and minerals, to be orally administered for the amelioration of the clinical parameters caused by the excess of body iron.

**Description**

Technical field

The present invention relates to compositions for treating iron overloading in human subjects comprising anthocyanins or oligomeric proanthocyanins or mixtures thereof in combination with lipophilic antioxidant vitamins. The administered orally compositions of the invention may be optionally in combination with common nutritional supplements.

Background of the invention

Iron overloading also known as hemosiderosis, siderosis, hemochromatosis, or chromatosis, is a relatively common condition leading to complicances due to oxidative stress catalyzed by excess body iron, causing relevant damages to body sites such as biomolecules (e.g. hormones), biological fluids (e.g. synovial fluid), tissues (e.g. liver, pancreas) and cardiovascular system, mainly to the heart causing arrythmias and heart failures.

The chronic treatment based on blood transfusions is usually mandatory in subjects with refractory anemia, thalassemia major, aplastic anemia, congenital (Blackfan-Diamond) anemia, acquired red cell aplasia, and may be necessary in subjects with diseases such as thalassemia intermedia, sickle cell anemia, and in myelodisplasia. The repeated transfusions cause iron overloading, which in turn is the origin of a number of clinical complicances.

Further iron overloading arise from non-transfusion dependent pathologies with increased iron deposition, i.e. thalassemia intermedia, sickle cell anemia, sideroblastic anemia, haemolytic anemia, as well as in alcoholic cirrhosis, porphyria cutanea tarda, and hydiophatic hemochromatosis.

Further iron overloading from non-transfusion dependent pathologies are neurodegenerative diseases such as Parkinson's disease, as pointed out by Lan J and Jiang DH in J Neural Transm, 104 (6-7): 649-60 (1997), Jellinger KA in Drugs Aging, 85(4):115-40 (1999) or Alzheimer's disease, as illustrated by Deibel MA; Ehmann WD; Markesbery WR in J Neurol Sci, 29(7):137-42 (1996).

The unbalanced iron condition leads to the progressive iron storage in different body compartments, a well-known clinical condition in transfusion-dependent subjects, which are systematically treated with iron chelators. On the other hand, the administration of drugs capable of iron depletion is far from being routinary on non transfusion-dependent iron overloading subjects.

A specific iron chelation therapy is presently provided by desferoxamine and deferiprone. These drugs remove iron from main targets, but, in transfusion-dependent subjects, cannot successfully decrease the residual iron content, since ferritin (as typical marker) usually remains in the range of 500-2.000 meg/ml in plasma, i.e. above the physiologic levels.

Moreover, only in the cirrhotic stage of primary forms of hemochromatosis, an increase of reticuloendothelial iron and serum ferritin occur, since both iron markers remain normal in the precirrhotic stage. Moreover, iron chelators should be administered continuously, so that the treatment of subjects with non transfusion-dependent iron overloading is usually not convenient.

The activity against iron overloading of some flavonoids, such as quercetin, diosmetin, kaempferol, hesperidin, green tea catechins or sylibin has been disclosed in Pietrangelo et al., Gastroenterology, 109 (6) 1941-49, 1995; Grinberg et al., Biochem. Pharmacol, 54 (9), 973-8, 1997; Afanas'Ev, Biochem. Pharmacol. 50 (5), 627-35, 1995; Morel et al., Biochem. Pharmacol. 45 (1) 1993, 13-19, 1903; Acker Van, et al., Biochem. Pharmacol, 56(8), 935-43, 1998; Ferroli M. et al. FEBS Letters, 416(2), 123-129, 1997; Wugno et al, Radiation Physics and Chemistry, 50(3), 271-6, 1997; Morel et al, Methods in Enzymology, vol. 234, 437-443, 1994.

Jørgensen L.V. and Skibsted L.H. in Free Radic Res, 58(1):335-51 (1998), evaluated of the relationship between redox potential and rate constants of the reduction of ferrylmyoglobin to metymyoglobin measured by cyclic voltammetry of the aforementioned compounds and of other flavonoids, such as myricetin, morin, taxifolin, chrysin, apigenin, luteolin, eryodictyol. The conclusion that higher oxidized Flavonoids turned out to be preferable than those having lower oxidized state.

Van Acker A.A., in Bioch. Pharmac., 56, 943-953, (1988), studied the inhibition of Fe-independent lipid peroxidation of several Flavonoids and compared with available data on the Cu-dependent lipoperoxidation, coming to similar conclusion.

However, these studies have been carried out on model systems which do not take in account the peculiar condition of iron overladoing, whereas the prevalence of high potency short-range oxygenated radicals generated from iron excess shall lead to different criteria for the selection of the most suitable flavonoids for the treatment thereof.

Description of the Invention

It has now been found that the combination of anthocyanins, or oligomeric proanthocyanins or mixtures thereof with lipophilic antioxidant vitamins is particularly advantageous for the treatment of iron overloading.

The present invention concerns therefore the use of anthocyanins and for oligomeric proanthocyanins in combination with lipophilic antioxidant vitamins for the preparation of medicaments for the treatment of iron overloading.

The present invention concerns also the compositions comprising anthocyanins, oligomeric proanthocyanins, or mixtures thereof in combination with lipophilic antioxidant vitamins and optionally other ingredients, useful for treating iron overloading occurring in transfusion-dependent and non-transfusion-dependent iron overloaded subjects.

It has been particularly found that the combination of anthocyanin or oligomeric proanthocyanins with lipophilic antioxidant vitamins such carotenoids, tocopherols and the like, produces a synergistic effect which can be advantageously used in the therapy of pathologies characterized by iron overloading.

Anthocyanins and oligomeric procenthocyanins, although belonging to the very wide family of flavonoids, differ from those already studied as possible agents form treating iron overload conditions, disclosed in the previously cited references, in several properties, making their choice unobvious and even contrary to the general teachings derivable from the prior art as a whole, suggesting the use of flavonoids having very high antioxidant potential.

Anthocyanins and oligomeric proanthocyanins, even if not the most active from the point of view of primary antioxidant potential, exhibit the following properties, which make them particularly suitable for the use according to he invention:

-   high biovailability after oral administration;
-   lack of mutagenic and cytotoxic activity;
-   radical scavenging activity particularly directed against short-range, high potency hydroxy radicals OH, i.e. against the radicals which are mostly involved in iron overloading;
-   stability to autooxidation in the presence of iron ions, and ability to retain secondary antioxidation activity by forming inert iron-complexes.

It will be apparent therefore that, while the primary antioxidant, (i.e. the direct radical scavenging activity is important, it is not sufficient in order to solve the therapeutic problem of iron overloading, the above properties, which have not been considered by the prior-art, being of paramount importance.

According to one of its aspects, the present invention thus concerns a dietetic and/or pharmaceutical composition comprising an effective amount of anthocyanins and oligomeric proanthocyanins with lipophilic antioxidant vitamins.

The oligomenc proanthocyanidins (OPC) comprise dimers, trimers, tetramers, or decamers of the repeated flavanols units, i.e. (+)-catechin and (-)-epicatechin. The OPC are commonly extracted and purified from grape seed, pine bark, cocoa and other vegetal sources rich in flavan-3-ols in the free, glucosylated, esterified or condensed forms.

Their inhibitory activity against biochemically generated superoxide anion and hydroxyl radical is 10 to 20 times higher than of that vitamin C and vitamin E, respectively.

Anthocyanins are present in the petals of flowers, in the leaves of most plants and in colored fruits and vegetables. These 2-phenylbenzopyrylium (flavylium) positively charged molecules bear hydroxy or methoxy groups (e.g. pelargonidin, cyanidin, delphinidin, petunidin, peonidin, malvidin), and may also be glycosylated as mono, di- and tri-saccharides, or acylated, e.g. with p-coumaric acid, in position 3.

Chalcones are the biosynthetic precursors of flavonoids and anthocyanins, the latter being slowly converted to the corresponding chalcones in neutral or slightly alcaline aqueous solution.

Anthocyanins and oligomeric proanthocyanidins are preferably extracted from grape and grape derivatives such as pomace, plum and seeds which also contain resveratrol and its condensed polymers, the viniferins.

A further preferred source of anthocyanins are blackberries, cranberries, strawberries, blueberries or other berry fruits.

The anthocyanins and proanthocyanins may be in form of aglycones, O-glycosides or naturally occurring ethers and esters thereof.

Suitable lipophilic antioxidant vitamins include tocopherols, carotenoids, lipoates and ubiquinones.

The $\alpha$-tocopherols (vitamin E), often associated with the $\beta$- and $\gamma$-isomers in most plants, are usually obtained from wheat and soybean oils.

Carotenoids, a family of are well known lipophilic antioxidant vitamins, include carotene and licophene.

Carotene in the form of $\alpha$-, $\beta$-, $\gamma$-, and $\delta$-isomers, known as provitamins A since they are converted into vitamin A by liver enzymes in the human body following the oral intake, as well as lycopene, are mainly found in tomatos, in carrots and palm oil, as well as in the green leaves of several plants.

Other carotenoids which may be used according to the invention include xantophylles, such as zeaxanthin, bixine, crocetin, criptoxanthin, rubixanthin, violaxanthin, fucoxanthin, lycophyll and lutein, found either in plants or in algae.

Ubiquinones, or coenzymes Q (Coenzyme $Q_6$ to Coenzyme $Q_{10}$), are a group of benzoquinonic substances, found in animals, plants and microorganisms.

The compositions of the invention may also comprise other nutritional supplements.

Examples of said nutritional supplements include:

- vitamins A and C.
- L-cysteine and its esters reduced or oxidized glutathione.
- Egg or plant phospholipids, i.e. phosphatidyl choline, phosphatidyl ethanolamine, phosphatidyl glucerol, phosphatidic acid, phosphatidyl inostitol, ceramides, and spyngolipids.
- Maltol and ethylmaltol.
- Oligoelements, i.e. Zn, Se, Mn, Cr, Mg, V, and other essential non-iron elements, as salts, oxides, chelated or complexed forms.
- Pro-antioxidant substances, e.g. vitamins $B_1$, $B_2$, PP, $B_6$, $B_{12}$, $K_1$, folic acid, pantothenic acid, biotine and the like, amino acids, alpha hydroxy acids, polyunsaturated fatty acids and conjugated linoleic acid.

Salts or complexes of magnesium, zinc and selenium are particularly preferred.

Maltol or ethylmaltol, commonly used as flavouring and fragrance-enhancing agent for foods, are also preferably added to the compositions of the invention. Maltol is particularly preferred.

The compositions of the invention preferably comprise at least 200 mg of anthocyanin or oligomeric proanthocyanins per unit dose and at least 100 mg of lipophilic antioxidant vitamins per unit dose.

The compositions of the invention are preferably administered by the oral route, in form of sachets, soft or hard gelatin capsules, fast or slow-release tablets or in liquid form.

The compositions of the present invention may also include non-toxic inert carrier or diluent in admixture with the above mentioned ingredients. Examples of such non-toxic, inert carriers include wheat starch, silica fumes, calcium carbonates, and sodium carboxymethyl cellulose.

The dose of anthocyanins and proanthocyanins, is typically from 1 to 20 mg/kg b. w. while the lipophilic antioxidant vitamins preferably subministered at the RDA levels.

The optimum dose of course depends on the body weight of the subject as well as the severity of iron overloading. Such a daily dosage of the compositions will give the subject the amount of iron sequestration and the inhibition of secondary oxidation, necessary to prevent iron damages.

The daily intake of 1 to 2 tablets or capsules, each containing 250 mg to 1000 mg of active the combination of anthocyanins and proanthocyanins with lipophilic antioxidant vitamins, is recommended for the treatment of iron overloading in transfused patients, e.g. in thalassemia major, aplastic anemia, congenital anemia, acquired red cell aplasia, myelodisplasia, and in optionally transfusion-dependent thalassemia intermedia and sickle cell anemia.

When iron overloading is a non transfusion dependent type of hemochromatosis or thalassemia intermedia, sickle cell anemia, sideroblastic anemia, haemolitic anemia, alcoholic cirrhosis, porphyria cutanea tarda, hidiophatic hemochromatosis, Parkinson's disease, and Alzheimer's disease, the compositions are preferably administered as tablets or capsules 1-3 times a day, each containing from 50 to 400 mg of the combination of anthocyanins and proanthocyanins with lipophilic antioxidant vitamins, preferably avoiding a total intake exceeding 500 mg per day.

There are no restrictions on age and duration of treatment.

The compositions may also be in form of free flowing bulk package or a of pre-dosed package, such as sachets to be admixed to food or drink, as well as dosed liquid supplements which contain all of the active ingredients of the dietary supplement in unit dosage form.

The following examples further illustrate the invention.

Example I - Retention of the secondary antioxidant activity

The oxidation resistance to an oxidative biological environment was testeed by applying an oxidative stress on few Flavonoids. The residual secondary antioxidant activity after pre.oxidation were misured in in vitro ex-vivo models and compared with the activity of non pre-oxidized compounds.

Pre-oxidation of flavonoids

1 g each of anthocyanins, proanthocyanins, quercetin and naringin were separately charged into flat-bottom conical flasks (500 ml). The flavonoids tested exhibits the following characteristics:

a) Anthocyanins with grape flavonoids (AGF) (4:1 mol/mol), content 50%; average MW = 338.7 (calculated as delphinidin); source grape pomace; from Enocianina Fornaciari Srl (Reggio Emilia, Italy).
b) Grape seed oligomeric proanthocyandins (GS-OPC); content $\geq$ 90%; average MW = 578 (calculated as A2-dimer); from Centroflora Ltd (Sao Paulo, Brazil).
c) Quercetin $2H_2O$; content 99%; MW = 338; from Sigma-Aldrich Srl (Milan, Italy).
d) Naringin; content $\geq$ 95% PM = 580.5; Freeman Industries Inc. (New York, NY, USA).

The flasks were charged with 100 ml of NaOH 0.1 N are equipped with an air pump (Silent Air[®], manufactured by Renn-Plax, Inc. in Taiwan R.O.C.) connected by a flexible hose to a disperser, which is placed at the bottom of the flask. The air is forced through the disperser and finely divided throghout the alkaline solution, kept under air bubbling for a time ranging from ½ hours at room temperature.

At the end of the reaction, the mixtures are acidified with HCl 3N until pH 5-6, and kept under stirring for about 1 hour. The pre-oxidized product obtained (a') from (a) was separated by freeze-drying; whilst the products (b'), (c') and (d') from the pre-oxidation of (b), (c) and (d), respectively, were recovered by filtering, washing with distilled water and dried under vacuum at mild temperature.

<u>Inhibition of Fe-induced lipoperoxidation on LDL by intact and pre-oxidized flavonoids</u>

The test was carried out by modification of the procedure according Visioli F. and Galli C. "Evaluating oxidation processes in relation to cardiovascular disease: a current review of oxidant/antioxidant methodology." in Nutr. Metab. Cardiovasc. Dis. 7: 459-466 (1997).

Briefly, human LDL (d=1.021-1.063) were isolated by sequential ultracentrifugation from plasma obtained from healthy, normolipidemic volunteers.

Before initiation of the experiments, LDL samples were desalted by size-exclusion chromatography and their protein content was determined according to Lowry, O., et al., J. Biol. Chem., 1951, 193, 265 (1951). The samples were prepared by diluting LDL in PBS at a final concentration of 200 μg protein/ml. The probes were thereby pre-incubated for 30 minutes at 37°C with 5 mg of the Flavonoids under investigation, or without any antioxidant (the "control"), then ferrous sulfate was added at 5 uM final concentration to start the oxidation, which was carried out at room temperature in a shaking bath. After 2 hours, samples were withdrawn for the quantitation of the tiobarbituric acid reacting substances (TBARS).

Results are listed in Table I.

TABLE I

| Inhibition of LDL oxidation by intact and pre-oxidized flavonoids | | | |
|---|---|---|---|
| | Lipoperoxidation (nM TBARS/ mg LDL) | Inhibition (%) | Differencial inhibition (%) (x-x'/x)*100 |
| control | 28.75 | - | |
| (a) | 14.89 | 48 % | |
| (a') | 11.68 | 59% | 22% |
| (b) | 8.73 | 70% | |
| (b') | 9.52 | 67% | -9% |
| (c) | 14.36 | 50% | |
| (c') | 24.85 | 14% | -73% |
| (d) | 22.41 | 22% | |
| (d') | 24.98 | 13% | -11% |

As it can be noted, the anthocyanins (a) after the short oxidation treatment (a') have an increase of their antioxidant activity, probably due to the formation of high chelating polydentate structure of the chalcones, thereby produced under alkaline conditions. The proanthocyanosides (b) exhibits the highest chelation activity, which is substantially mantained after pre-oxidative treatment (b'). Of the comparative flavonoids tested, quercetin (c) undergoes a significant degradation of the capacity to inhibit iron-dependent lipoperoxidation after oxidation (c'); whilst naringin (d) shows a modest oxidative inhibition, which is slightly decreased by the pre-oxidation treatment (d').

Is therefrom confirmed that the prevention of the formation of the short-range $OH^\circ$ in system with prevalence of Fenton-like reactions is better performed and retained by anthocyanins or proanthocyanidins even when a previous oxidative stress shall occur.

Example II - Gastro-resistant tablets

Gastro-resistant tablets of with an extract of anthocyanins 150 mg and proanthocyanidins 100 mg, both from Vitis vinifera, were prepared in the usual way with the following ingredients:

| | |
|---|---|
| microcrystalline cellulose | 118 mg |
| precipitated silica | 3 mg |
| magnesium stearate | 4 mg |
| anionic polymer of methacrylic acid and its esters | 12 mg |
| talc | 8 mg |
| magnesium carbonate | 8 mg |
| maize starch | 5 mg |
| gum arabic | 159 mg |
| anthocyanins | 150 mg |
| proanthocyanidins | 100 mg |
| vitamin E (a-tocopherol) | 20 mg |
| carotenoids | 1 mg |

Example III - Carbonated beverage

A beverage syrup is made in the usual way by mixing the following ingredients:

| | |
|---|---|
| distilled water | 9,444 g |
| phosphoric acid (75%) | 50 g |
| citric acid | 8 g |
| cherry flavor | 186 g |
| liquid fructose (77% by weight solids) | 8,483 g |
| sucrose | 726 g |
| $\alpha$-tocopherol | 10 g |
| anthocyanins | 70 g |
| proanthocyanidins | 53 g |

This syrup is mixed with carbonated water to prepare a carbonated cherry-like beverage: 117.3 g of syrup is diluted with 353.5 ml of carbonated water at a level of 3.2 to 3.4 volumes of carbon dioxide.

Example IV - Fast release capsules with anthocyanins

A composition is prepared in the usual way in form of capsule with the following ingredients:

| anthocyanins | 400 mg |
|---|---|
| calcium carbonate | 250 mg |
| magnesium hydroxide | 160 mg |
| zinc subcarbonate | 350 mg |
| ethyl maltol | 50 mg |
| alpha-tocopherol | 12 mg |
| lipoic acid | 15 mg |
| $\alpha$-tocopherol | 6 mg |

with the balance a nutritionally acceptable carrier.

Example V - Fast release capsules with proanthocyanidins

A composition is prepared with the ingredients of Example II except that 400 mg of anthocyanins are replaced by 250 mg of proanthocyanidins.

Example VI - Hydroalcoholic composition

A composition is prepared in form of ampoules with the following ingredients:

| anthocyanin | 400 mg |
|---|---|
| ascorbic acid | 12 mg |
| maltol | 30 mg |
| L-tartaric acid | 15 mg |
| strawberry flavor | 2 mg |
| $\alpha$-tocopherol | 12 mg |
| lipoic acid | 15 mg |
| coenzyme Q10 | 3 mg |

solubilizing the mixture in ethyl alcohol 35° qs to 250 ml.

Example VII and Comparative Example I - Polyvitamins and mineral composition comprising anthocyanins and proanthocyanidins vs quercetin

The following compositions are prepared in the form of a 1250 mg capsule comprising the ingredients listed in the left column. For comparison, a commercially available composition for general purpose anti-oxidant protection, namely the "Super Anti-oxidant" manufactured by The Vitamine Shoppe® (N. Bergen, NJ, USA), is listed in the right column.

|  | Example VIII | Comparative Example I |
|---|---|---|
| vitamin A and β-carotene | 0,967 mg | 5,000 IU |
| vitamin E | 30 mg | 100 IU |
| α-lipoic acid | 10 mg | 5 mg |
| coenzyme Q10 | 5 mg | 5 mg |
| garlic extract | - | 50 mg |
| vitamin K1 | 17.5 mcg | - |
| vitamin C | 45 mg | 30 mg |
| vitamin B1 | 0.35 mg | 12.5 mg |
| vitamin B2 | 0.4 mg | 12.5 mg |
| vitamin B3 | - | 12.5 mg |
| vitamin B5 | - | 12.5 mg |
| vitamin B6 | 0.5 mg | 12.5 mg |
| vitamin B12 | 0.75 mcg | 125 mcg |
| vitamin PP | 4.5 mg | - |
| folic acid | 50 mcg | - |
| biotin | 0.038 mg | - |
| pantothenic acid | 1.5 mg | 12.5 mg |
| N-acetyl cysteine | - | 25 mg |
| glutathione | - | 25 mg |
| magnesium | 180 mg | - |
| manganese | 5 mg | 7.5 mg |
| selenium | 27.5 mcg | 37.5 mcg |
| zinc | 15 mg | 15 mg |
| maltol | 50 g | - |
| anthocyanins | 100 mg | - |
| proanthocyanidins | 50 mg | 5 mg |
| quercetin | - | 12.5 mg |
| flavanols from green tea | - | 3.75 mg |

with the balance a nutritionally acceptable carrier.

Comparative clinical trial

16 patients affected by homozigous β-thalassemia major, actually receiving approximately 15 ml of packed red blood cells per kg body weight at interval of of 2-4 weeks, were recruited and divided in two groups of 8 individuals. They were instructed to take regularly at least 1 tablet per day of the composition of Example VII (group A) and Comparative Example I (group B), respectively, for a period of 3 months. They were also recommendation to assume the product with water or other soft drink, possibly at fast and not associated with dairy products.
The hematological data of the two groups of transfusion dependent subjects before and after a three months treatment, i.e. with compositions of Example VII (group A) and Comparative Example I (group B), are listed in Table II.

TABLE II

| | Group A | | Group B | |
|---|---|---|---|---|
| | initial | end | initial | end |
| iron (mg/dl) | 254+/-60 | 223+/-71 | 249+/-84 | 257+/-93 |
| ferritin (ng/ml) | 1866+/-996 | 1798+/-891 | 1732+/-705 | 1803+/-944 |
| NTBI (µg/dl)* | 21.8+/-13.8 | 18.9+/-15.1 | 20.7+/-12.5 | 19.4+/-16.1 |
| TIBC (µgFe/l)** | 369+/-97 | 373+/-40 | 355+/-50 | 341+/-109 |
| aspartate transaminase (U/l) | 81.5+/-33 | 57.1+/-19 | 80.7+/-25 | 65.8+/-29 |
| alanine transaminase (U/l) | 86.5+/-35 | 63.2+/-22 | 81.7+/-25 | 75.8+/-39 |
| malondialdehyde (nM/ml) | 2.10+/-0.77 | 1.54+/-0.98 | 2.06+/-0.94 | 1.99+/-0.83 |
| conjugated dienes (nM/ml) | 13.4+/-2.3 | 7.1+/-5.1 | 12.9+/-2.8 | 11.5+/-6.7 |
| protein carbonyls (nM/ml) | 1.52+/-0.59 | 0.80+/-0.56 | 1.74+/-0.41 | 1.02+/-0.84 |
| urate (µM/l) | 330+/-95 | 283+/-30 | 318+/-40 | 305+/-81 |
| ascorbate (µM/l) | 30.1+/-10.6 | 43.4+/-8.7 | 38.7+/-15.1 | 42.8+/-18.8 |
| bilirubin (mM/l) | 1.36+/-0.34 | 0.88+/-0.19 | 1.28+/-0.25 | 1.01+/-0.39 |
| vitamin E (µM/l) | 10.9+/-4.2 | 13.8+/-5.0 | 11.3+/-3.5 | 11.0+/-4.6 |
| vitamin A (µM/l) | 1.01+/-0.30 | 1.49+/-0.13 | 1.19+/-0.24 | 1.18+/-0.29 |
| β-carotene (µM/l) | 0.30+/-0.07 | 0.42+/-0.05 | 0.32+/-0.10 | 0.34+/-0.29 |
| lycopene (µM/l) | 0.20+/-0.08 | 0.28+/-0.11 | 0.23+/-0.14 | 0.31+/-0.19 |

NTBI (µg/dl)* = non transferrin bound iron, measured in 500 µl serum samples by the method of
Singh S. et al., (Anal. Biochem. 186:320 1990), based on the colorimetric reaction with bathfenanthroline, as modified by Zhang D. et al., Bioch. Mol. Biol. Int. 35:635 (1995);
TIBC (µgFe/l)** = total iron binding capacity, evaluated by the commercial kit from Sigma (St.
Louis, MO, USA).

As it can be noted, the group A treated with the composition comprising anthocyanins and proanthocyanidins was significantly superior as regard to a similar composition comprising quercetin. The parameters of liver functionality, aspartate transaminase (AST) and alanine transaminase (ALT) were significantly improuved, as well as bilirubin, thus indicating that hemolysis at the red blood cell was partially prevented
The content of endogenous antioxidants is significantly increased in both groups, however the better trend of conjugated dienes, malondialdehyde and protein carbonyl indicates a better lipoperoxidation inhibition, thus with a decreased content of fragmented or oxidized material from the apoptic cell due to the oxidative stress.

**Claims**

1. Use of anthocyanins and oligomeric proanthocyanins in combination with lipophilic antioxidant vitamins for the manufacture of a medicament for the treatment of iron overloading.

2. Use according to claim 1 wherein said anthocyanins are selected from pelargonidin, cyanidin, deiphinidin, petunidin, peonidin, malvidin, and mixture thereof.

3. Use according to claim 1 wherein said proanthocyanins are condensed oligomers consisting of cyanidin (+)-catechin and (-)-epicatechin units.

4. Use according to claim 2 or 3 wherein said anthocyanins and oligomeric proanthocyanins are in form of O-glycosides, and naturally occurring esters and ethers thereof.

5. Use according to claims 2 or 3 wherein said lipophilic antioxidant vitamins are selected from tocopherols, carotenoids, ubiquinones, lipoates and mixtures thereof.

6. Use according to claim 1 wherein said iron overloading derives from blood transfusion therapy.

7. Use according to claim 1 wherein said iron overloading derives from non transfusion-dependent thalassemia intermedia, sickle cell anemia, sideroblastic anemia, haemolytic anemia, alcoholic cirrhosis, porphyria cutanea tarda, and hidiophatic hemochromatosis.

8. Use according to claim 1 wherein said iron overloading derives from non transfusion-dependent iron overloading neurodegeneration.

9. Pharmaceutical or nutritional compositions comprising anthocyanins, oligomeric proanthocyanins or mixtures thereof and lipophilic antioxidant vitamins.

10. Compositions according to claim 9 for oral administration in solid form or in liquid form.

11. Compositions according to claims 9 or 10 further comprising magnesium, zinc, selenium salts or complexes.

12. Compositions according to claim 11 comprising magnesium salts or complexes.

13. Compositions according to any one of claims 9 to 12, further comprising maltol an/or ethylmaltol.